# EUROPEAN PATENT APPLICATION

(11) **EP 3 069 658 A1**
(43) Date of publication of application: **21.09.2016**
(21) Application number: 16161240.3
(22) Date of filing: 18.03.2016
(51) Int. Cl.: A61B 5/113, A61B 5/08, A61B 5/00

(54) **SENSOR DEVICE FOR DETECTING DISPLACEMENTS OF HUMAN'S BODY SURFACE ACCOMPANYING RESPIRATION**

(30) Priority: 20.03.2015 JP 2015057916
(71) Applicant: Toyota Jidosha Kabushiki Kaisha, Toyota-shi, Aichi 471-8571 (JP); UEDA JAPAN RADIO CO., LTD., Ueda-shi, Nagano 386-8608 (JP)
(72) Inventor: HATAKEYAMA, Yoshiyuki, Aichi-ken, Aichi 471-8571 (JP); SHIGETO, Kazuhide, Aichi-ken, Aichi 471-8571 (JP); KIKUCHI, Hirokazu, Aichi-ken, Aichi 471-8571 (JP); OHYA, Shigemasa, Ueda-shi, Nagano 3868608 (JP); NAITOU, Tukasa, Ueda-shi, Nagano 3868608 (JP); TAKAHASHI, Hironobu, Ueda-shi, Nagano 3868608 (JP); SUZUKI, Takahiro, Ueda-shi, Nagano 3868608 (JP)
(74) Representative: D Young & Co LLP

(57) **Abstract**

In a sensor device which detects pressure variations owing to displacements of a body surface of a subject by respiration through variations of the output impedance of a piezoelectric vibrator, the pressure variations owing to displacements can be more efficiently transmitted to the piezoelectric vibrator. The sensor device, attached to a subject's body surface and detecting displacements of the body surface accompanying respiration of the subject, comprises a contact piece having a contact surface which contacts the body surface when the sensor device is attached to the body surface; a displacement-signal convertor which is connected with the contact piece and converts the displacements of the body surface into electrical signals; and an outputting device which outputs the electrical signals to an external unit; wherein the contact surface of the contact piece is a curved surface projecting outward in a direction from its periphery to its center.

## Description

### Technical field

This invention relates to a device which detects displacements of a body surface accompanying human's respiration, and more specifically to a sensor device which is attached to a body surface under pressure in the direction of suppressing the displacements of the body surface accompanying respiration and detects pressure variations owing to the displacements of the body surface while converting them into electric signals.

### Background art

The mental or physiological conditions, relating to various feelings and consciousness, such as concentration, stress, strain, rest, awakening, sleepiness, sleep, etc., arise by activities of a brain and/or nerves of a human being (or an animal) (biological conditions), and these are reflected in the conditions of respiration or cardiac beats. Then, in recent years, in the field of technique for detecting or estimating the biological condition of a person as described above, it has been tried to estimate a biological condition as described above through the measuring of respiration and/or cardiac beats. Especially, in one way of measuring a condition of respiration or cardiac beats as noted, a sensor device is attached to a body surface under pressure in the direction of suppressing displacements of the body surface accompanying respiration or cardiac beats, whereby the pressure variations owing to the displacements of the body surface, converted into electric signals, are detected. For instance, in patent documents 1 and 2 of a part of applicants of the present application, there have been proposed a device and a method of measuring pressure variations owing to displacements of a body surface of an animal by respiration or cardiac beats in time series by utilizing the phenomenon that the impedance of a piezoelectric vibrator, which vibrates by exerting alternating voltage at a natural resonance frequency while being pressurized onto the body surface of an animal, varies with the pressure variations owing to the displacements of the body surface of an animal by respiration or cardiac beats. Further, Japanese Design Application No. 2014-8970 discloses a design of such a sensor device to be pressured and attached onto a body surface of an animal.

### Prior technical documents

### Patent documents

Patent document 1: WO2013/111785
Patent document 2: JP2015-20033

### Summary of Invention

### Technical Problem

In a sensor device (a wearable sensor device) attached to a subject's body surface in a device which measures pressure variations owing to displacements of the subject's body surface by respiration or cardiac beats as described in the above-mentioned patent documents 1 and 2, it is preferable that its output, i.e., the impedance of a piezoelectric vibrator, (output impedance) is substantially proportional to the pressure variations owing to the displacements of the body surface while the pressure variations owing to displacements of the body surface is also measurable as a value as large as possible. In this respect, in patent document 2, a contact piece, made of comparatively flexible rubber elastic material, is interposed between a piezoelectric vibrator and a subject's body surface to be compressively deformable when the piezoelectric vibrator is pushed toward the subject's body surface, whereby it can be realized to make the output impedance increase almost linearly to the input, i.e. the pressure variations owing to displacements of the body surface. However, for example, the pressure measured at a body surface of a waist or an abdomen, corresponding to displacements of the diaphragm of a human being by respiration, is a minute pressure at the level of 0.1 N/cm², and also, can become still lower depending on a posture, a physique and a wearing garment of the subject, while the resolution of 0.015 (N/digit) is required in order to establish that the normalization correlation value with waveforms obtained in a respiratory band, approved as a medical machine, becomes 0.9 or more for the requirements for a respiratory sensor device. Therefore, it is desirable to transmit pressure variations owing to displacements of a body surface to a piezoelectric vibrator still more efficiently to obtain much larger variations of the output impedance.

Thus, one object of the present invention is to make it possible to more efficiently transmit pressure variations owing to displacements of a body surface of a subject (human being) by respiration to a piezoelectric vibrator in a sensor device which detects the pressure variations owing to displacements of the body surface with the output impedance variations of the piezoelectric vibrator.

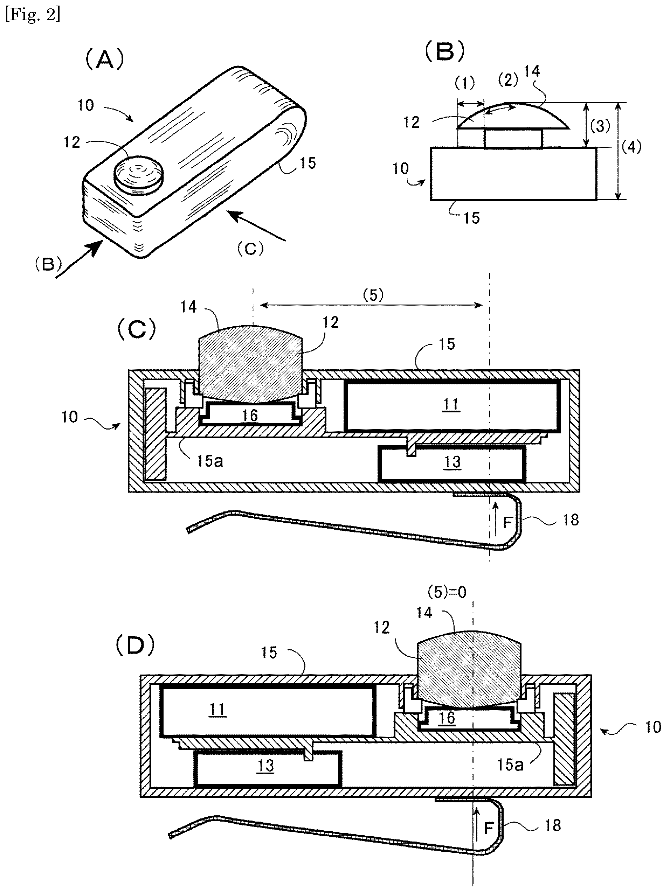

With respect to the above-mentioned object, the inventors of the present invention have conducted investigations for the shape of a contact piece arranged between a piezoelectric vibrator and a site to be tested on a body surface and the position of a contact piece in a sensor device in order to increase pressure variations (input pressure variations) given to a piezoelectric vibrator from the motion of a body surface. In the investigations, more concretely, the inventors have examined conditions and/or structures, appropriate for the increase of input pressure variations at the piezoelectric vibrator to the pressure variations by displacement of a body surface, with respect to (1) the umbrella width of a contact piece; (2) the shape of the contact surface of a contact piece; (3) the head height; (4) the total height of a sensor device; and (5) the arrangement between a contact piece and a fastener (a stopping member or a clip) for attaching a sensor device with a subject's body surface, as schematically drawn in Fig. 2B. In the present invention, the advantageous conditions for increase of the input pressure variations at a piezoelectric vibrator have been found out especially for (1) the umbrella width of a contact piece and (2) the shape of the contact surface of a contact piece, and the validities of the conditions have been confirmed through the verification experiments by experimental design method.

Thus, a more detailed object of the present invention is to provide such a device which detects motion waveforms on a subject's body surface by respiration by the output impedance variations of the piezoelectric vibrator, the device satisfying conditions of the umbrella width and/or the contact surface shape of a contact piece, advantageous for increasing the input pressure variations owing to displacements of the body surface.

### Solution to Problem

According to the present invention, the above-mentioned object is achieved by a sensor device, attached to a body surface of a subject and detecting displacements of the body surface accompanying respiration of the subject, comprising: a contact piece having a contact surface which contacts the body surface when the sensor device is attached to the body surface; a displacement-signal convertor which is linked to the contact piece and converts the displacements of the body surface into electrical signals; an outputting device which outputs the electrical signal to an external unit, wherein the contact surface of the contact piece is a curved surface projecting outward in a direction from its periphery to its center. In this structure, as noted above, the displacement-signal convertor may be typically a piezoelectric vibrator of which an impedance varies with pressure variations owing to displacements of the body surface of the subject by respiration when alternating voltage at its natural resonance frequency is applied to it under a condition that it is contacted under pressure onto the subject's body surface, but may not be limited thereto, and it may be an arbitrary means to convert pressure variations owing to displacement of the subject's body surface into electrical signals. Further, in the above-mentioned structure, typically, the displacement-signal convertor and outputting device are accommodated in a chassis or a housing and the contact piece is fixedly arranged on a working surface of the displacement-signal convertor, e.g., the back of one electrode in a case of a piezoelectric vibrator.

In a case that the contact surface of the contact piece is formed in a curved surface, projecting in the direction from its periphery to its center as noted above, when the contact piece is pushed on a subject's body surface, the subject's body surface is deformed concavely to abut along the center region of the contact surface projecting in a curved surface because of its flexibility. Then, the degree of the close adherence between the contact surface and the body surface increases so that the pressure variations owing to displacements of the body surface will be transmitted to the contact piece stably (namely, under a condition with little influence of the posture, physique, wearing condition of the subject), and therefore, there will be expected an increase of the input of the displacement-signal convertor to the pressure variations owing to displacements of the body surface. Moreover, because of the cured shape of the contact surface, even if the angle of the contact surface to the body surface is changed with the change of the posture, physique, wearing condition, etc. of the subject, no large change of its contact area occurs, and thereby it is expected that the stable transmission of the pressure variations owing to displacements of the body surface to the contact piece is maintained. In this regard, as explained in detail in the column of Embodiments mentioned later, preferably, it has been found out that the radius of curvature of the contact surface of a contact piece may be between 7 mm and 50 mm. Moreover, in order to increase the input variations to the pressure variations owing to displacements of the body surface, preferably, the contact piece may be formed with a material which is not substantially deformed by the pressure variations owing to displacements of the body surface.

Further, in the structure of the above-mentioned inventive sensor device, preferably, the height of the contact piece and the total height of the sensor device each are set such that, when the sensor device is attached to the subject's body surface while being clamped between the subject's body surface and garment, the contact piece is pushed onto a subject's body surface so that the body surface will be deformed concavely along the contact surface. In a case that the sensor device is attached to the subject's body surface and the displacements of the body surface are detected thereon, if the contact area between the contact piece and body surface varies with a change of the posture, physique, wearing condition, etc. of the subject, the transmitted amount of the pressure variations owing to displacements of the body surface to the contact piece can be changed. Thus, in the present invention, as noted above, the sensor device is designed to be clamped between a body surface and a garment of the subject, and further, the contact piece and the sensor device may be constructed such that the height of the contact piece and the total height of the sensor device each have a height and a total height such that the contact piece is pushed under pressure onto a body surface so that the body surface will be deformed concavely along the contact surface. According to this structure, even if the posture, physique, wearing condition, etc. of the subject is changed at a certain degree, the sensor device remains held between the concavely deformed body surface and the garment so that the position of the contact surface to the body surface will not be easily shifted, and thereby, the variation in the contact area between the contact piece and the body surface is suppressed as small as possible, and it is expected that the pressure variations owing to displacements of the body surface can be stably captured. In this regard, since there is a possibility that a subject will feel discomfort if the forcing of the contact piece to the body surface is too strong, it is preferable that the height of the contact piece and the total height of the sensor device are adjusted at a degree that a subject could not feel discomfort. In this respect, as explained in detail in the column of Embodiments mentioned later, preferably, it has been found out that a preferable range of the total height of the sensor device is from 16 mm to 23 mm and a more preferable range of the height of the contact piece is from 3 to 5 mm.

Furthermore, in the above-mentioned inventive structure, the sensor device may be clamped between the body surface of the subject and the upper edge portion of a bottom garment which the subject wears, wherein the contact piece may be put on one surface of the housing which accommodates the displacement-signal convertor and outputting device while a stopping member which can be attached with a upper edge portion of a bottom garment may be provided on the surface of the other side of the housing. Here, "bottom garment" is a garment for the lower body of a subject, such as trousers, a skirt, and a hakama, having the upper edge portion extending along the circumference of the subject's waist. The stopping member may be a clip member which cooperates with the surface of the other side of the housing to clamp the upper edge portion of a bottom garment in between them. The stopping member or clip member may be attached to the upper edge portion of a bottom garment while being hung down from a belt or a band wound around the upper edge portion.

According to the above-mentioned structure, it is advantageous in that the attaching of the sensor device to a subject's body surface is achievable only by inserting the sensor device between the subject's body surface and the upper edge portion of a bottom garment without using a specialized band for fixation which should be wound around the circumference of a subject's body trunk. Also, the sensor device is clamped between a subject's body surface and the upper edge portion of a bottom garment so that the forcing of the contact surface of the contact piece to the body surface will be achieved, and thus, especially, in the structure of hanging and hooking a stopping member or a clip member to the upper edge portion of a bottom garment, even when the distance between the upper edge portion of a bottom garment and a body surface increases due to a subject's posture change, it can be avoided that the sensor device drops out of the upper edge portion of the bottom garment. In addition, it also becomes possible to force the contact surface of the contact piece onto a body surface at an appropriate pressure only by adjusting the tightening of a belt or a band.

### Effect of Invention

Generally, according to the structure of the present invention, the adherence property of the contact surface of the contact piece of the sensor device and a subject's body surface is improved, and accordingly, even if the change of the direction of the sensor device occurs due to the change of the posture, physique, wearing condition, etc. of the subject, the shifting of the sensor device (the moving of its position) and/or a large change of the contact area between the contact surface of the contact piece and the body surface are suppressed, and thereby, it is expected to attain stable detection of the pressure variations owing to displacements of the body surface by respiration. In this regard, the validities of the inventive characteristic structures explained above have been verified in the experiments explained later. Further, as already noted, although the present invention is explained about with respect to the device which detects pressure variations owing to displacements of a body surface of a subject by respiration with the variations in an output impedance of a piezoelectric vibrator, the present invention is expected to be useful in a sensor device using an arbitrary means to covert pressure variations owing to displacements of a subject's body surface into electrical signals, and thus, it should be understood that such cases belong to the scope of the present invention, also.

Other purposes and advantages of the present inventions will become clear by explanations of the following preferable embodiments of the present invention.

### Brief Descriptions of Drawings

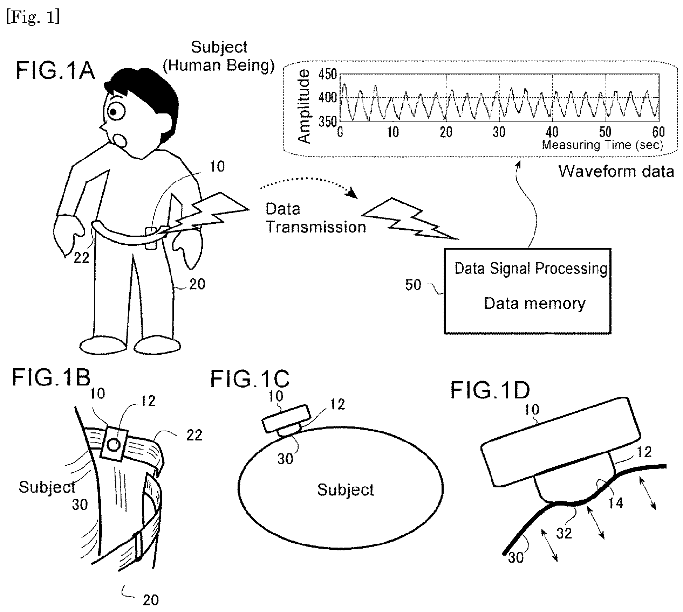

Fig. 1A is a drawing explaining the overview of a system of a device measuring pressure variations owing to displacements of a body surface accompanying respiration of a human being, to which a sensor device according to the present invention is applied. Fig. 1B is a drawing showing a manner of attaching the inventive sensor device to the upper edge portion of a subject's trousers (bottom garment). Fig. 1C is a drawing showing schematically an appearance of the inventive sensor device being contacted to the body surface of a subject's waist. Fig. 1D shows schematically an appearance of the contact piece of the inventive sensor device which is contacted onto a subject waist body surface under pressure.
Fig. 2A is a typical perspective diagram of a sensor device according to the present invention. Fig. 2B is a side view of the sensor device seen in the direction of arrow (B), explaining about parameters in the shapes of a contact piece and a sensor device to be examined for the increasing of the input pressure variations owing to displacements of a body surface. Figs. 2C and 2D is sectional views of the inventive sensor device seen in the direction of arrow (C). Fig. 2C shows a case where an offset is provided between a contact piece and a force-applied point of a clip, and Fig. 2D shows a case where no offset is provided between a contact piece and a force-applied point of a clip.

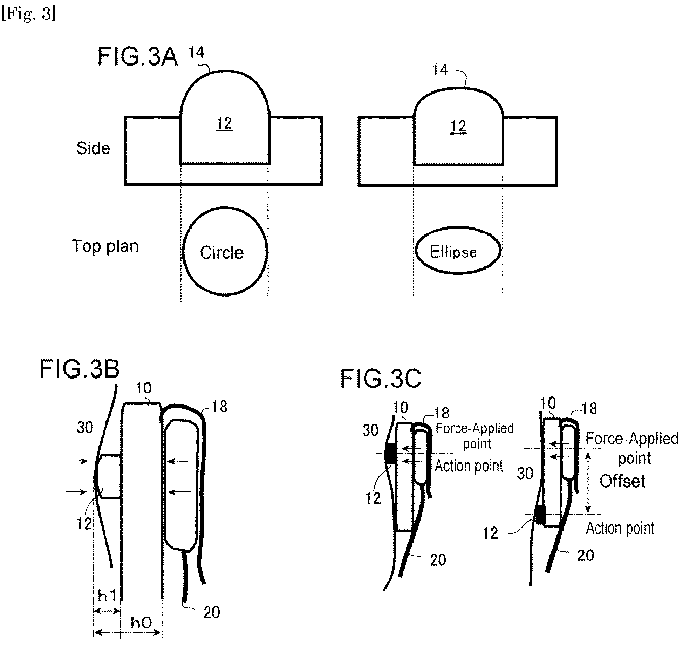

Fig. 3A is a drawing showing schematically the shape of a contact surface of a contact piece which can be used for the inventive sensor device. Fig. 3B is a drawing explaining about a condition of the inventive sensor device clamped between a subject's body surface and the upper edge portion of trousers. Fig. 3C explains about the positions on which the contact piece is attached in a case that no offset is provided between a contact piece and a force-applied point of a clip (left) and a case that an offset is provided between a contact piece and a force-applied point of a clip (right).

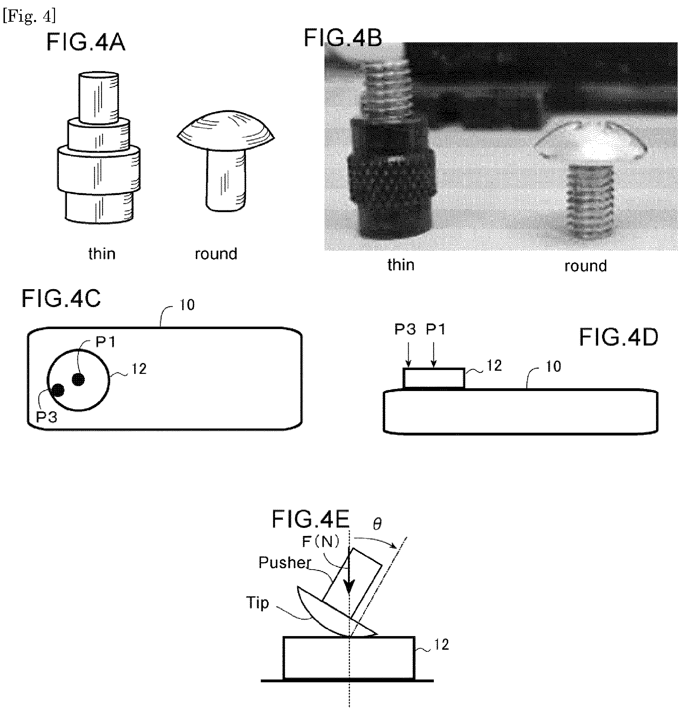

Figs. 4A and 4B show schematic drawings and photographs of pushers, having been used in the experiments for verification of conditions of shapes of a contact surface of a contact piece of a sensor device. Figs. 4C and 4D schematically show a top plan view and a side view of a sensor device to which the pusher was contacted in the experiments for verification of conditions of shapes of a contact surface of a contact piece. Fig. 4E is a side view of a contact piece and a pusher showing the condition of forcing the pusher on the contact piece of the sensor device.

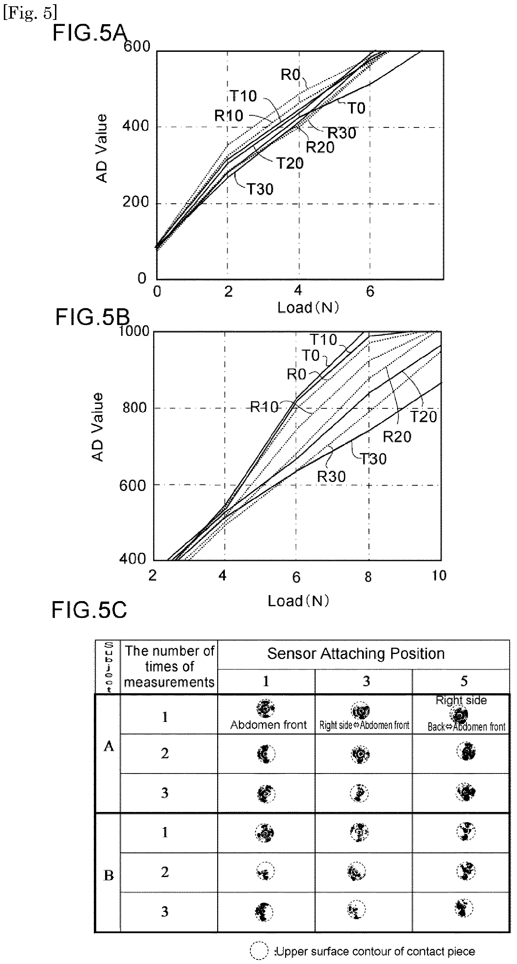

Fig. 5A is a graph chart showing variations of the output value of a sensor device to the load (AD converted value of the impedance of a piezoelectric vibrator) in the cases of forcing the pusher on the position P1 in the contact piece of Figs. 4C and 4D, and
Fig. 5B is a graph chart showing variations of the output value of a sensor device to the load (AD converted value of the impedance of a piezoelectric vibrator) in the cases of forcing the pusher on the position P3 in the contact piece of Figs. 4C and 4D. Fig. 5C shows regions stained with ink on subject's sites, i.e., regions where a contact surface actually contacted, when a contact piece having a flat contact surface to which ink had been applied were forced on various sites of a subject.

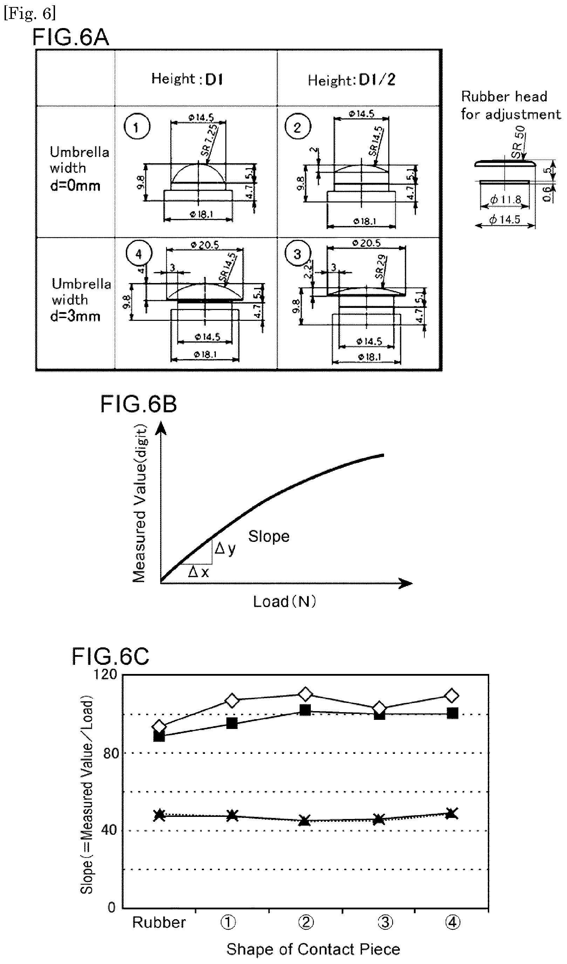

Fig. 6A shows side views showing the sizes of contact pieces of various shapes used in the verification experiments for checking the validities of conditions of the shapes of the contact piece and the sensor device according to the present invention. Fig. 6B is a drawing showing schematically a variation of the output value of a sensor device when a load is applied on a contact piece in Fig. 6A while changing the value of the load. Fig. 6C is a graph chart indicating slopes of the output values of the sensor device to the load at a contact piece in the measuring of Fig. 6B, using the contact pieces of various shapes in Fig. 6A.

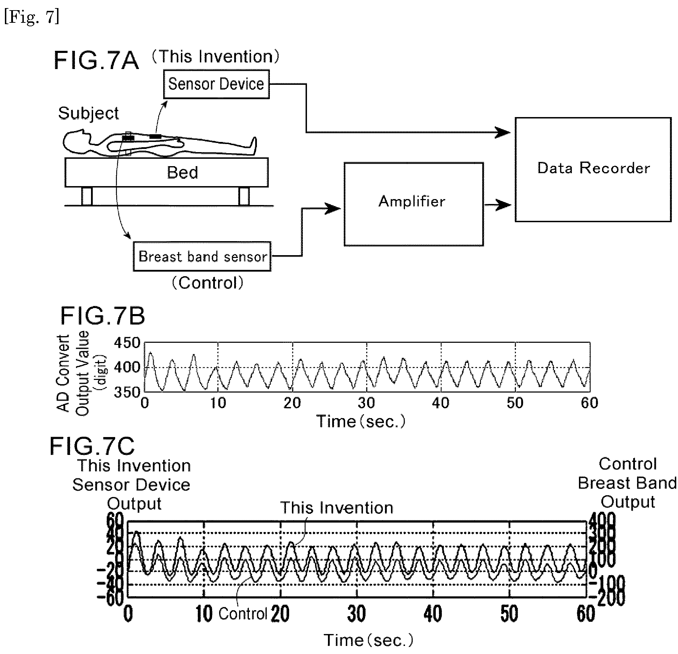

Fig. 7A shows a schematic diagram of a system for measuring pressure variations owing to displacements of a body surface accompanying a subject's respiration, using a sensor device attached to a subject's waist according to the present invention and a respiratory sensor of breast band type attached to a subject's chest (for control). Fig. 7B shows time variations of the output value (AD converted value of the impedance of a piezoelectric vibrator) of the sensor device according to the present invention. Fig. 7C shows time variations of the output value of the sensor device according to the present invention (this invention) and time variations of the output value of the breast band type respiratory sensor (control) (in the illustrated data, the amplitudes have been adjusted for comparison after a noise removing process.). In this regard, for the contact piece of the sensor device according to the present invention, a contact piece having 20 mm of total height and 4 mm of head height was used.

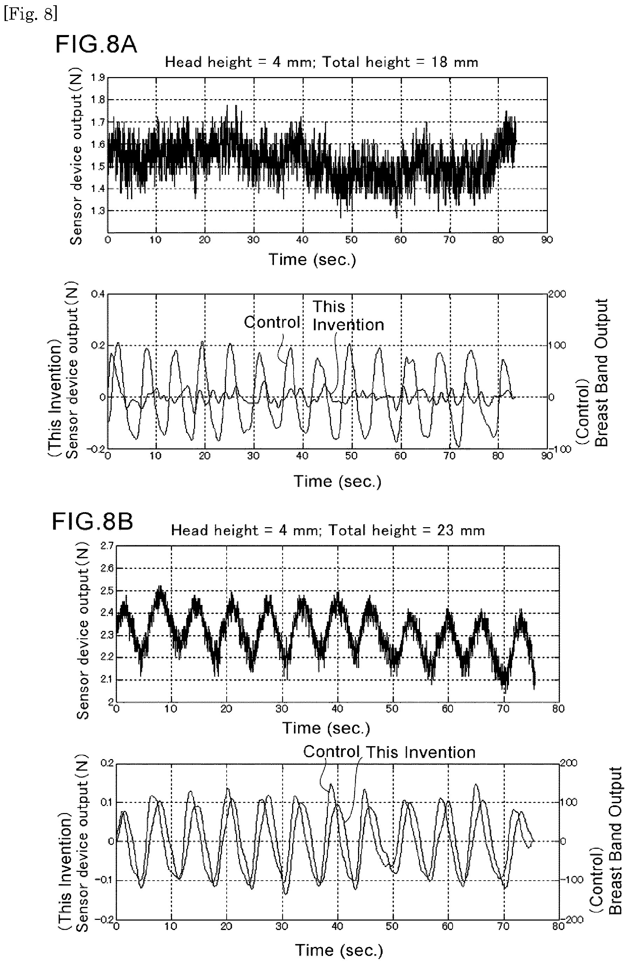

Figs. 8A and 8B show time variations of the output values of the sensor device according to the present invention and the breast band type respiratory sensor in measurements conducted similarly in Fig. 7A. Fig. 8A shows results in using a contact piece, having 18 mm of total height and 4 mm of head height, of the sensor device according to the present invention, where the upper row shows an intact waveform obtained by converting the output value of the inventive sensor device in force unit, and the lower row shows waveforms, after noise removal, of time variations of the output values of the inventive sensor device (This invention) and the breast band type respiratory sensor (Control). Fig. 8B shows results in using a contact piece, having 23 mm of total height and 4 mm of head height, of the sensor device according to the present invention, where the upper row shows an intact waveform obtained by converting the output value of the inventive sensor device in force unit, and the lower row shows waveforms, after noise removal, of time variations of the output values of the inventive sensor device (This invention) and the breast band type respiratory sensor (Control).

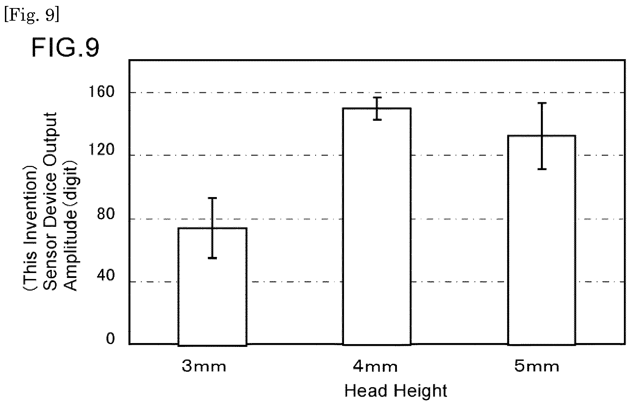

Fig. 9 shows amplitudes of the waveforms of the output values of the sensor device using various head heights for the contact piece in measurements conducted similarly in Fig. 7A.

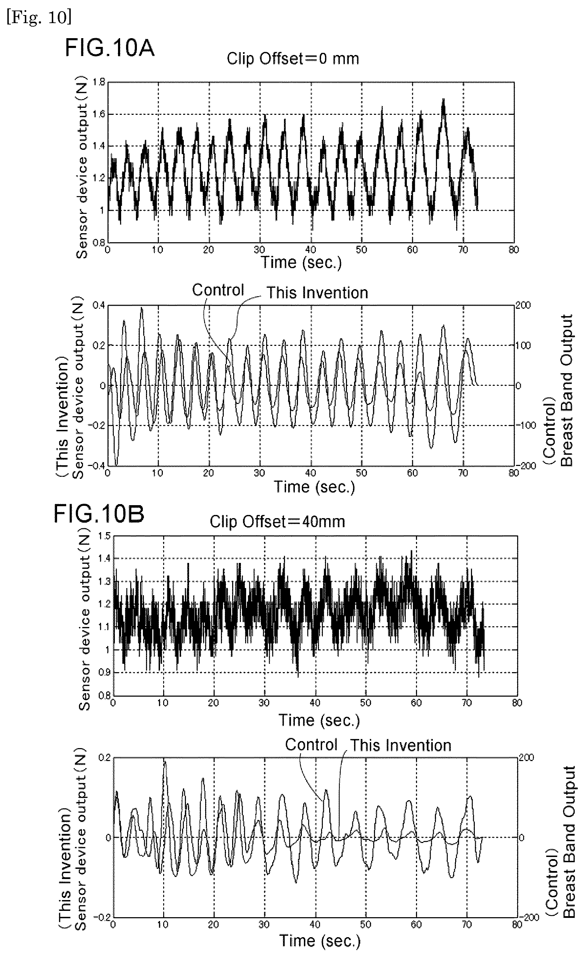

Figs. 10A and 10B show time variations of the output values of the sensor device according to the present invention and the breast band type respiratory sensor in measurements conducted similarly in Fig. 7A. Fig. 10A shows results in a case of setting the offset between a contact piece and a force applied point to be 0cm in the sensor device according to the present invention, where the upper row shows an intact waveform obtained by converting the output value of the inventive sensor device in force unit, and the lower row shows waveforms, after noise removal, of time variations of the output values of the inventive sensor device (This invention) and the breast band type respiratory sensor (Control). Fig. 10B shows results in a case of setting the offset between a contact piece and a force applied point to be 4cm in the sensor device according to the present invention, where the upper row shows an intact waveform obtained by converting the output value of the inventive sensor device in force unit, and the lower row shows waveforms, after noise removal, of time variations of the output values of the inventive sensor device (This invention) and the breast band type respiratory sensor (Control). In the experiments, a contact piece having 20 mm of total height and 4 mm of head height was used.

### Explanations of Reference Numerals

- 10: Sensor device
- 11: Communication apparatus
- 12: Contact piece
- 13: Battery
- 14: Contact surface of a contact piece
- 15: Sensor device housing
- 16: Piezoelectric vibrator
- 18: Clip
- 20: Trousers
- 22: Upper edge portion of trousers
- 30: Body surface of a subject
- 32: Site of the body surface abutting on a contact piece
- 50: Data signal processor

### Description of Embodiments

In the followings, preferable embodiments of the present invention are described in detail. In the drawings, the same reference numerals indicate the same sites.

### Overview of Respiratory Waveform Measuring System

The sensor device, which detects displacements of a body surface accompanying respiration of a human being, in accordance with the present invention is used as a part of a system for measuring respiratory waveforms of a human being as schematically drawn in Fig. 1A. In the respiratory waveform measuring system as illustrated, briefly, a sensor device 10, attached with a body of a human being, measures, in time series, displacements of the body surface moving in vibration by respiration or pressure variations owing to the displacements; transmits its measured values to a data signal processor 50 through radio or cable communication; and generates time series waveform data of respiratory motion as drawn in the upper row in the drawing. As already noted, since the respiratory motion waveform data relates to biological conditions in connection with activities of the brain or nerves of a human being (or an animal), estimation of a biological condition will be performed with the respiratory motion waveform data.

For the sensor device attached to a body of a human being in the respiratory waveform measuring system as described above, there has been employed in the past a type of device in which an instrument measuring displacements of a body surface is wound and tighten around the whole circumference of a subject's chest, etc. by a breast band. However, this type of device is slightly large-scale as a sensor device, and it takes time and effort to attach the sensor device on a subject's body surface. Therefore, in the present embodiments, as illustrated, there is employed a sensor device which is relatively small in size and attachable to an upper edge portion, a belt or a band 22 of a garment for a lower half of a subject's body, such as trousers 20 (bottom garment).

### Structure of Sensor Device

As schematically drawn in Fig. 2A, typically, the above-mentioned sensor device 10 has a housing 15 of several centimeters in size with a contact piece 12 projecting outward, and the sensor device 10 is hung and hooked on the upper edge portion, belt or band 22 of a subject's bottom garment 20 with a clip 18 (a clamping hook - see Fig. 2C) attached to the side opposite to that of the contact piece 12, as schematically illustrated in Fig. 1B, and further pushed on a body surface 30 of a subject's abdomen - waist so that the contact piece 12 projecting from the housing will be pressurized onto the body surface 30 as in Fig. 1C. Then, when the body surface 30 of the abdomen - waist is displaced owing to the subject's diaphragm by respiration, as indicated by arrows in Fig. 1D, variations in pressure which the contact piece 12 receives occur by the displacements, and thus, the pressure variations are converted into electrical signals, transmitted to the data signal processor 50 and measured as index values representing respiratory motions. For the conversion from the pressure variations to electrical signals, although various principles may be employed, especially the present invention employs the principle utilizing the phenomenon that, when a piezoelectric vibrator, contacted via a contact piece on a body surface under pressure, vibrates by applying alternating voltage at its natural resonance frequency, the impedance of the piezoelectric vibrator varies with pressure variations that it receives through the contact piece (Patent documents 1 and 2). In this case, in the inside of the sensor device 10, more concretely, as shown in Fig. 2C while being partially simplified (Fig. 2D shows the same structure although its direction is reversed.), a piezoelectric vibrator 16 is held by a support frame 15a in the housing 15, and the contact piece 12 is arranged such that a contact surface 14 projecting in one side of the contact piece 12 from the housing 15 abuts on the subject's body surface while the other side of the contact piece 12 abuts on to the piezoelectric vibrator 16. Then, to the piezoelectric vibrator 16, alternating voltage is applied at its natural resonance frequency through an electric circuit, not illustrated, from the power supply 13, and the variations of the impedance of the piezoelectric vibrator 16 owing to pressure variations that the contact piece 12 receives are digitized, and transmitted to the data signal processor 50 with communication apparatus 11 in time series.

With respect to the sensor device 10 as described above, as noted in the column of "Summary of Invention", in order to measure the pressure variations owing to displacements of a body surface with more sufficient sensitivity, it is required to transmit the pressure variations owing to displacements of a body surface to the piezoelectric vibrator 16 as efficiently as possible. As noted, the pressure measured on a body surface of a waist or an abdomen, corresponding to the movement of the diaphragm of a human being by respiration, is a minute pressure at the level of 0.1 N/cm², and also, in the case of the sensor device attached to a waist or an abdomen, the transmission performance of pressure variations can become still lower due to changes of the contact area and/or pressing force between the body surface and the contact piece, depending upon the posture, physique, wearing condition, etc. of a subject. Therefore, in the sensor device attached to a waist or an abdomen as described above, it is desirable to improve the shape and/or arrangement of the contact piece so as to make the transmission of the pressure variations owing to displacements of a body surface to the piezoelectric vibrator 16 better.

Then, the inventors of the present invention have examined conditions and structures in the sensor device 10 as mentioned above for achieving the increase of the input pressure variations at a piezoelectric vibrator to pressure variations owing to displacements of a body surface, namely, the improvement in the transmission performance of pressure variations to a piezoelectric vibrator, with respect to the following five structural requirements as shown in Figs. 2B, 2C and 2D: (1) the umbrella width of a contact piece 12, (2) the umbrella top curvature, (3) the head height,(4) the sensor device total height, and (5) the arrangement of a contact piece and a clip for attaching a sensor device to a subject's body surface, and the validities of those conditions were confirmed by the verification experiments in accordance with the experimental design method.

Thus, in the present invention, by designing the sensor device 10 and the contact piece 12 so that the following conditions or structures for the above-mentioned five structural requirements may be filled, the increase of the input pressure variations at a piezoelectric vibrator to pressure variations owing to displacements of a body surface will be achieved. (The results of the verification experiments in accordance with the experimental design method will be mentioned later.)

With respect to (1) the umbrella width of a contact piece 12, namely, the length of the umbrella-like portion of the upper part of a contact piece projecting from the perimeter of the lower cylindrical portion in the radial direction (see (1) in Fig. 2B), it has been found that a shape projecting by significant length (about 2-3 mm), namely, a shape with an umbrella is more advantageous than a shape whose perimeter of the upper umbrella-like portion coincides with the perimeter of a lower cylindrical portion (a shape whose projecting length is 0 - a shape without umbrella) according to the results (not shown) of the variance analysis in the verification experiment. However, there were found no significant differences between a shape with an umbrella and a shape without umbrella in the slopes of the input pressure variations at a piezoelectric vibrator to the pressure variations owing to displacements of a body surface. Therefore, it was found out that the umbrella-like portion of the upper part of a contact piece needs not project outward from the perimeter of the lower cylindrical portion in the radial direction.

With respect to (2) the shape of the contact surface 14 of a contact piece 12, namely, the shape of the surface contacting a subject's body surface in the upper surface of a contact piece (see Fig. 2B (2)), it is considered that, as compared with a flat contact surface, a contact surface having a curved surface projecting in the direction from the periphery to the center as schematically drawn in Fig. 3A can achieve better transmission of pressure variations from a body surface 30 to a piezoelectric vibrator 16 because the larger contact area between the curved contact surface 14 and the body surface 30 is obtained when the body surface 30 is deformed concavely by pressing the curved contact surface 14 of the contact piece 12 onto the body surface 30 as schematically drawn in Fig. 1D, and also, the contact area will not easily changed even in an occurrence of the mutual directional change between the body surface 30 and the contact surface 14. The shape seen from the projecting direction of the contact piece 12 (plan view) may be a perfect circle or may be an ellipse. In this regard, in order to increase the variations in the input to pressure variations owing to displacements of a body surface, namely, in order to transmit more efficiently the pressure variations owing to displacements of a body surface to a piezoelectric vibrator, preferably, a contact piece is made of material which is not substantially deformed by the pressure variations owing to displacements of a body surface. According to the results of the verification experiments, it has been found out that a curved contact surface is more advantageous than a flat contact surface because the slope of the input pressure variations at a piezoelectric vibrator to the pressure variations becomes larger and the dynamic range also becomes larger in the curved contact surface. Furthermore, in accordance with the results of the verification experiments, it was found out that the input pressure variations at a piezoelectric vibrator to pressure variations can be detected advantageously when the radius of curvature of a contact surface is from 7 to 50 mm.

With respect to (3) the head height, namely the height of a contact piece 12 projecting from the upper surface of a housing 15 of a sensor device 10 (see Fig. 2B (3)), and (4) the total height of a sensor device, namely, the height of a contact piece 12 from the bottom of a housing 15 of the sensor device 10 (see Fig. 2B (4)), in the structure which clamps a sensor device 10 between a subject's body surface 30 and the upper edge portion 22 of a bottom garment 20 as noted above, it is preferable that the contact surface of the contact piece 12 is pressed onto the body surface at a moderate pressure so that the change of the contact area between the contact piece and the body surface will be suppressed as much as possible even when any change of the posture, physique, wearing condition, etc. of the subject occurs in order to transmit pressure variations owing to displacements of the body surface efficiently to a piezoelectric vibrator 16. For this purpose, as drawn in Fig. 3B, preferably, the head height h1 and the total height h0 of the sensor device 10 are designed such that the contact piece 12 will be pushed on the body surface 30 under pressure, and in the direction opposite to this, the body surface pushes the contact surface while being deformed concavely along the contact surface. However, if the head height h1 and the total height h0 are too large so that the forcing of the contact piece 12 to the body surface 30 is too strong, a subject could feel discomfort. Then, according to the verification experiments, it has been found out that, for the conditions of (3) the head height and (4) the total height of a sensor device satisfying the above-mentioned demands, a preferable range of the total height of the sensor device is from 16 mm to 23 mm and a more preferable range of the height of the contact piece is from 3 mm to 5 mm.

With respect to (5) the arrangement of a contact piece and a clip, in the structure of the present invention, as shown in Fig. 2C, a sensor device 10 is clamped between a body surface and the upper edge portion 22 of a subject's bottom garment 20 while a clip 18 is hung and hooked on the upper edge portion 22, and thereby, a force F exerted by the upper edge portion 22 (for example, a force exerted by the tightening of a belt or a band) acts from the contact surface 14 of the contact piece 12 so that the sensor device 10 will be attached on the subject's body surface 30 under the condition that the contact surface 14 is pressed on the body surface 30. In this respect, as shown in Fig. 2D, in a case that the contact surface 14 of the contact piece 12 exists on the extension line (alternate long and short dash line) of the site on which the force F acts

(force-applied point), as in Fig. 3C left, the body surface 30 will be deformed concavely by forcing the contact piece 12 to the body surface 30 so that the contact piece 12 will be satisfactorily held between the body surface 30 and the upper edge portion 22 of the bottom garment 20 under pressure. However, as shown in Fig. 2C, in a case that the position of the contact surface 14 of the contact piece 12 deviates (offset) from the extension line (alternate long and short dash line) of the force-applied point, the contact piece 12 may not be satisfactorily held between the body surface 30 and the upper edge portion 22 of the bottom garment 20 under pressure, depending upon the length of its offset, as in Fig. 3C right. Thus, in the present invention, basically, a clip is arranged on a sensor device 10 such that the contact surface 14 of the contact piece 12 will be positioned on the extension of the point F on which the upper edge portion 22 exerts the force. In this regard, according to the verification experiments, it was found out that the distance (clip offset) between the force applied point F of a clip and the contact surface 14 of a contact piece 12 needs not exactly be 0 mm, but may be around 40 mm. It should be understood that such a case belongs to the scope of the present invention, also.

### Verification of Conditions for Structures of Sensor Device and Contact Piece

With respect to conditions for the above-mentioned five structural requirements, those validities were confirmed by verification experiments in accordance with the experimental design method.

### 1. With respect to Conditions of preparing the contact surface of a contact piece into a curved surface

### (Experiment 1)

Referring to Figs. 4A - E, the output of a sensor device 10 was measured by pushing a pusher (thin, round) having a shape as shown in Figs. 4A and 4B onto the sensor device 10 having a contact piece 12 having a flat upper surface as schematically drawn in Figs. 4C and 4D. The pusher was placed on a position P1 (almost center) or a position P3 (near periphery) on the contact piece 12 as shown in Figs. 4C and 4D while its tip was directed downward and inclined at an angle θ (0° - 30°) as shown in Fig. 4E, and then, a load F (0-14N) was applied from the top of the pusher onto the contact surface.

Fig. 5A shows variations of the outputs of the sensor device 10 in applying the load F with the pushers "thin" and the pusher "round", positioned on the position P1 of the contact piece 12, respectively. In the graph chart, R0, R10, R20 and R30 (all indicated in dotted lines) show results in the cases of setting the pusher "round" at the angle θ of 0°, 10°, 20° and 30°, respectively, and T0, T10, T20 and T30 (all indicated in solid lines) show results in the cases of setting the pusher "thin" at the angle θ of 0°, 10°, 20° and 30°, respectively. The axis of ordinate indicates AD converted value of the output of a sensor device. With reference to the drawing, when changing a load from 0 to 4N, the rising of the output is larger in the pusher, "round", showing that it is more advantageous. Fig. 5B shows the results in conducting the same experiments in the position P3 of the contact piece 12. With reference to the drawing, in the case of the position P3, although no significant differences between the pusher "thin" and the pusher "round" and no significant differences depending upon the angles were not seen when the loads from 0 to 4N were given (not shown), the saturation of the output occurred at an early stage in the pusher "thin" when the angle θ was small (R0, T0, R10, T10). That is, it has been shown that the dynamic range of the pusher "round" is larger than that of the pusher "thin".

### (Experiment 2)

In a sensor device 10 having a contact piece 12 with a flat contact surface (upper surface), ink was applied on the upper surface (contact surface) and the contact surface to which the ink had been applied was attached on a subject's abdomen or back, and then a mark (trace) of the ink remaining on the body surface was observed. Fig. 5C shows the ink traces which remained in the body surfaces when contact pieces were attached onto the abdomen front; between the abdomen front and the right side; and the right side in subjects A and B, respectively. In a plurality of times of the attaching (1-3), there were observed no examples in which ink trace remained on a site corresponding to the whole region of the upper surface of the contact in any case. Since the presence of a region where no ink traces are left suggests that the contact surface does not satisfactorily contact that region, it can be estimated that, in a case of a flat shaped contact surface, there exists a region which does not satisfactorily contact a body surface, and in such a region, the transmission of pressure variations is not good.

### (Experiment 3)

Using a sensor device equipped with a contact piece having a curved contact surface shown in Fig. 6A, its output was measured while a load of 0-15N was given to the contact surface of the contact piece, and, the slope of the output value of the sensor device to the load was computed as schematically drawn in Fig. 6B in each condition. The illustrated contact pieces (1)-(4) were made of a material which has a rigidity at a degree that no substantial deformation occurred even if it was pushed against a body surface, while the radii of curvature of the contact surfaces were (1)7.25mm, (2)14.5mm, (3)29mm, and (4)14.5mm, respectively, and, the radius of curvature of the contact surface of the contact piece made of rubber (rubber head for adjustment) was 50 mm. The umbrella widths of the rubber head for adjustment and the contact pieces (1) and (2) were 0 mm. Fig. 6C shows the slopes (Δy/Δx) of the output values to the loads in conducting measurements in which the contact piece (1)-(4) and the rubber head for adjustment were set on four different sensor devices, respectively. The respective plots (■, ◊, ×, ▲) indicate the results using mutually different device. "Rubber", (1), (2), (3), and (4) on the horizontal axis correspond to the cases that the contact pieces in Fig. 6A were used, respectively. With reference to this drawing, in the slopes of output values, there is no big difference in the slopes of output values depending upon contact pieces although there were individual differences depending upon the devices, and in the cases of the radii of curvature of 7-50 mm, the measurements were possible and no differences owing to the presence or absence of umbrella width were not observed. Moreover, although not in all the sensor devices, there was a tendency for the slope to be larger in the contact pieces having a rigidity (1) - (4) as compared with the contact piece made of rubber.

Thus, the results of the above-mentioned experiments 1-3 show that the rising-up of the output value to the load and/or the dynamic range are better in the cases of contact pieces with a curved contact face (Experiment 1), and that a region where a contact surface cannot fully contact a body surface occurs in a case of a flat contact piece (experiment 2), suggesting that better transmission of pressure variations will be achieved in a case of a contact piece having a curved contact surface. Also, for a contact piece having a curved contact surface, it has been suggested that the detection of pressure variations is possible when its radius of curvature is about 7-50 mm while the presence or absence of umbrella width hardly influences the result.

### 2. With respect to Conditions for Head Height of Contact Piece and Total Height of Sensor Device

Referring to Fig. 7A, in order to conduct the verification for the conditions of the head height of a contact piece and the total height of a sensor device, as illustrated, a sensor device 10 according to the present invention was attached on the abdomen of a subject laying on his back on a bed, and the outputs of the sensor device accompanying respiration of the subject were measured. In this regard, for controlled comparison, measurements by a respiratory measuring method using a sensor device of breast band type of which medical reliability has been confirmed were carried out simultaneously. In the measurements, the output of the sensor device 10 according to the present invention and the output of the output of the breast band type sensor device, amplified with an amplifier, were transmitted to a data recorder, and time series respiratory waveform data were generated.

Fig. 7B shows time series data of the output value of the inventive sensor device 10 with a contact piece having a curved contact surface where there were head height = 4mm, the total height =20mm, measured under a condition of the clip offset =0mm, and Fig. 7C is graph charts representing the respective time series data of the output value of the inventive sensor device 10 and the output value of the breast band type sensor device, where amplitudes have been adjusted for comparison. As understood with reference to the drawing, the time series data of the output value of the inventive sensor device 10 and the output value of the breast band type sensor device exhibited substantially, mutually synchronized oscillatory waveforms. The cross correlation coefficient between them computed out after normalizing the respective wave amplitudes into the range of 0-1 was 0.97. This value satisfies the demand that the normalized correlation with the waveform of a respiratory band approved as a medical machine should be 0.9 or more as described in "Summary of Invention".

Figs. 8A and 8B show results measured similarly to the above using sensor devices with the total height =18mm and 23 mm, respectively. Those cross correlation coefficients were 0.52 and 0.96, respectively. In the case of Fig. 8B, the demand that the normalization correlation value with the waveform of a respiratory band approved as a medical machine should be 0.9 or more is satisfied. In the case of Fig. 8A, the above-mentioned demand for the normalization correlation value is not satisfied, but the output waveform of the inventive sensor device generally synchronized with the output waveform of the breast band type sensor device.

Fig. 9 shows the average values of wave amplitudes of the output values of sensor devices 10 with the total height = 23 mm, obtained by measurements similar to the above while the head height was 3 mm, 4 mm, and 5 mm, respectively (Error bars are standard deviations.). In all the cases, significant measurements were possible.

Thus, it was shown that significant measurements are possible when the total height of a sensor device is in the range of 16 to 23 mm, and the height of a contact piece was in the range of 3 to 5 mm.

### 3. With respect to Conditions of Clip Offset

In the cases that the clip offset was 0 mm and 40 mm, subject's respiratory waveforms were measured using the inventive sensor device 10 similarly to the measurement experiments explained in Fig. 7A. Figs. 10A and 10B show time series data of the output values of the inventive sensor device 10 (upper row), and time series data of the output values of the inventive sensor device 10 and the output values of the breast band type sensor device, where the amplitudes were adjusted, in the cases of the clip offset = 0 mm, and = 40 mm, respectively. Those cross correlation coefficients were 0.93 and 0.55, respectively. In the case of Fig. 10A, the demand that the normalization correlation value with the waveform of a respiratory band approved as a medical machine are 0.9 or more is satisfied. In the case of Fig. 10B, the above-mentioned demand for the normalization correlation value is not satisfied, but the output waveform of the inventive sensor device generally synchronized with the output waveform of the breast band type sensor device. Thus, it was shown that significant measurements are possible when a clip offset is in the range of 0-40 mm.

Thus, as described above, it has been shown that measurements of subject's respiratory waveforms are possible in the inventive sensor device under the conditions for the above-mentioned five structural requirements.

Although the above explanation has been described with respect to embodiments of the present invention, it will be apparent for those skilled in the art that various modifications and changes are possible, and that the present invention is not limited to the above-illustrated embodiments and may be applied to various devices and apparatus without deviating from the concepts of the present invention.

## Claims

1. A sensor device, attached to a body surface of a subject and detecting displacements of the body surface accompanying respiration of the subject, comprising:
a contact piece having a contact surface which contacts the body surface when the sensor device is attached to the body surface;
a displacement-signal convertor which is linked to the contact piece and converts the displacements of the body surface into electrical signals; and
an outputting device which outputs the electrical signals to an external unit;
wherein the contact surface of the contact piece is a curved surface projecting outward in a direction from its periphery to its center.

2. The sensor device of Claim 1, wherein a radius of curvature of the contact surface of the contact piece is between 7 mm and 50 mm.

3. The sensor device of Claim 1, wherein the contact piece is made of material which does not deform substantially to the displacements of the body surface.

4. The sensor device of Claim 1, wherein a height of the contact piece and a total height of the sensor device each are set such that, when the sensor device is attached to the body surface while being clamped between the body surface and a garment of the subject, the contact piece is pushed onto the body surface so that the body surface will be deformed concavely along the contact surface.

5. The sensor device of Claim 4, wherein the total height is in the range between 16 mm and 23 mm.

6. The sensor device of Claim 5, wherein the height of the contact piece is in the range between 3 mm and 5 mm.

7. The sensor device of Claim 1, wherein the sensor device is clamped between the body surface of the subject and an upper edge portion of a bottom garment which the subject wears, and the contact piece is arranged in one side surface of a housing accommodating the displacement-signal convertor and the outputting device and a stopping member which can be attached with the upper edge portion of the bottom garment in the other side surface of the housing.

8. The sensor device of Claim 7, wherein the stopping member is a clip member which cooperates with the other side surface of the housing to clamp the upper edge portion of the bottom garment in between the clip member and the other side surface of the housing.
